# EUROPÄISCHE PATENTANMELDUNG

(11) **EP 1 123 707 A2**
(43) Veröffentlichungstag der Anmeldung: **16.08.2001**
(21) Anmeldenummer: 00127337.4
(22) Anmeldetag: 13.12.2000
(51) Int. Cl.: A61K 35/407, A61P 31/12

(54) **Verwendung eines Leberextraktes und Leberextrakt dazu**

(30) Priorität: 15.12.1999 DE 19960669
(71) Anmelder: Gruner, Helmut, Dr.med.vet, 61669 Friedberg (DE)
(72) Erfinder: Gruner, Helmut, Dr.med.vet, 61669 Friedberg (DE)
(74) Vertreter: Schlagwein, Udo, Dipl.-Ing.

(57) **Zusammenfassung**

Die Erfindung betrifft die Verwendung eines Leberextraktes und/oder Fraktionen desselben als Virustherapeutikum (universelles Immunstimulans), als virales Antiinfektiosum und zur Therapie von durch Prionen induzierten Erkrankungen. Bei dem Leberextrakt handelt es sich vorzugs-weise um ein Lebervollextrakt in Form eines Hydrolysates. Ihm kann ein Konservierungsstoff wie m-Kresol zugesetzt sein.

## Beschreibung

Die Erfindung betrifft Verwendungen eines Leberextraktes und ein Leberextrakt dazu.

Leberextrakt in Form eines Hydrolysates aus Rinderleber wurde in der Vergangenheit bei Menschen zur Therapie von Anämica perniciosa verwendet. Deshalb ist dessen Herstellung bereits bekannt, wird jedoch derzeit nicht mehr produziert und therapeutisch genutzt.

Sowohl beim Menschen als auch bei Tieren breiteten sich in letzter Zeit zunehmend Krankheiten aus, welche durch Viren oder Prionen verursacht sind und für die zur Zeit keine wirksamen Therapiemöglichkeiten bestehen. Der Erfindung liegt deshalb das Problem zugrunde, neue Verwendungen für Leberextrakt und fraktionierte Leberextrakte aufzuzeigen, durch die sich neue Therapiemöglichkeiten ergeben.

Eine erfindungsgemäße Lösung dieses Problems besteht in der Verwendung eines Leberextraktes und/oder Fraktionen desselben als Virustherapeutikum (universelles Immunstimulans).

Der Erfinder konnte durch Therapie von erkrankten Tieren mit einem solchen Leberextrakt bisher unbekannte Wirkungen des in der Leberextraktion vorhandenen therapeutischen Prinzips feststellen. Die Wirkung des Leberextraktes beruht nicht nur auf der Wirkung als allgemeines Immunstimulans, sondern vor allem auf der Tatsache, dass es aufgrund der oftmals geradezu frappierenden Wirkung als ein virales Antiinfektiosem aufzufassen ist.

Für die therapeutische Nutzung der in dem Extrakt enthaltenen Wirksubstanzen - herstellungsbedingt handelt es sich um ein Konglomerat natürlicher, biologischer Molekülkomplexe - ist die uneingeschränkte Wirksamkeit sowohl gegen DNA- wie RNA-Viren.

Der therapeutische Einsatz des Wirkstoffkomplexes erfolgt als intramuskuläre Injektion und variiert nur im Behandlungsschema. Akute bis subakute virale Infektionen sind meist mit 1 bis 3 Injektionen, und zwar im Ein-Tage-Intervall, zu beherrschen.

Subakute und chronisch verlaufende virale Infektionen erfordern naturgemäß eine sich über einen längeren Zeitraum erstreckende Therapie. Die Anfangstherapie erfolgt kurzzeitig im Ein-Tage-Intervall - später Übergang auf 1/2 bis Einwochenintervall (im Einzelfall auch längere Intervalle).

Als Dosisrichtwert gilt im Regelfall 0,08 ml des Therapeutikums je kg Körpermasse.

Nebenwirkungen sind nach eigener Erfahrung ausgesprochen selten und zählen zur großen Kategorie der Überempfindlichkeitsreaktionen.

Kontraindikationen: bei bekannter Allergie vs Rindereiweiß und absolute Kontraindikation - "Praekoma und Coma hepaticum".

Eine weitere erfindungsgemäße Therapiemöglichkeit besteht in der Verwendung eines Leberextraktes zur Therapie von durch Prionen induzierten Erkrankungen. Die Prionenwirksamkeit könnte vor allem durch eine spezielle Applikationsart zusätzlich potenziert werden.

Vor allem bei der Behandlung von durch Prionen induzierten Erkrankungen ist es vorteilhaft, wenn das Leberextrakt peridural appliziert wird. Die peridurale Applikation des Therapeutikums umgeht die Blut-Hirn-Schranke. Infolge der über den Periduralraum direkten Verbindung zum zentralen Nervensystem kann ein direkter Angriff des Therapeutikums auf Prionen im Hirn erfolgen. Bezüglich der Möglichkeit der Anwendung eines Therapeutikums durch eine peridurale Applikation verfügt der Anmelder auf dem Gebiet der Tiermedizin über eine große Erfahrung. Mit dieser Methode verabreichte er schon über viele Jahre übergeordnete Sexualhormone. Auf diesem speziellen Gebiet ist die peridurale Applikation allen anderen Verabreichungsarten in jeder Hinsicht überlegen.

Das Leberextrakt ist sehr einfach herstellbar, wenn es sich um ein Hydrolysat handelt.

Besonders wirksam ist das Leberextrakt, wenn es sich um ein Lebervollextrakt handelt.

Besonders vorteilhaft ist es, wenn das Leberextrakt einen Konservierungsstoff wie m-Kresol enthält. Für die vorgesehene Anwendung besteht das Therapeutikum aus 0,5 ml Leberextrakt in 1 ml Lösung. Der Anteil des m-Kresols beträgt 0,003 ml/ml Lösung.

Das erfindungsgemäße Leberextrakt kann auch eingesetzt werden, wenn es in homöopathischen Dilutionen für spezielle Indikationen (peridurale Injektion) verwendet wird.

Die geprüfte antivirale Wirkung des erfindungsgemäßen Therapeutikums nur an tierpathogenen Viren ergibt sich zwingend aus der beruflichen Tätigkeit des Anmelders als praktischer Tierarzt. Die Verwendung von Leberextrakt bei bestimmten relevanten Tierseuchen wurde von dem Anmelder einmal bei der Behandlung zweier im Frühstadium an Schweinepest erkrankter Mastschweine mit Erfolg erprobt.

Die Auswahl der Behandlung von Viruserkrankungen im Kleintierbereich rührt, was die Häufigkeit der Behandlung von Hcc.-Erkrankungen des Hundes betrifft, daher, weil der Anmelder das Therapeutikum erstmalig bei dieser Infektion eingesetzt hat. Zum Zweiten war das Therapeutikum zu diesem Zeitpunkt noch auf dem Markt. Andere weniger häufig behandelte Virusinfektionen fanden statt, als das Mittel schon teilweise vom Markt war und nur noch eigene Restbestände vorrätig waren. Zum Dritten lassen sich die Ergebnisse prinzipiell auf die Humanmedizin übertragen, da im Infektionsmodus und -verlauf viraler Erkrankungen bei Mensch und Tier kein Unterschied besteht.

Zur weiteren Verdeutlichung der Wirksamkeit des Leberextraktes wird nachfolgend über Therapiemaßnahmen des Anmelders berichtet:

### 1. Durch DNA-Viren verursachte Erkrankungen

### 1.1 Tierart: Hund

### 1.1.1 Hepatitis infectiosa canis (Hcc)

Diese Krankheit gehört zu den Kinderkrankheiten des Hundes, verläuft im akuten Krankheitsfalle unter dem klinischen Bild einer heftigen Gastroenteritis. Die spezielle Therapie mit Leberextrakt führt innerhalb von 36 - 48 Stunden zur Heilung. Alle vom Anmelder über Jahre therapierten Tiere (insgesamt 220) wurden gesund.

Die selten auftretende subakut verlaufende Form der Erkrankung - insgesamt 3 beobachtete Fälle - ist unheilbar. Alle diese Fälle waren von anderen Tierärzten vortherapiert. Obwohl virusindiziert, ist die Leberzirrhose aufgrund ihrer Pathogenese unabhängig von einem noch vorhandenen Virus durch völligen Ausfall der Leberfunktion für den tödlichen Ausgang der Erkrankung verantwortlich.

### 1.1.2 Parvovirose des Hundes (Gruppe der Parvoviren)

Insgesamt 3 behandelte Fälle. Alle in akutem Stadium der Erkrankung behandelten Hunde wurden ohne weitere zusätzliche Therapie mit zwei Injektionen geheilt. (3. Fall nur eine Injektion, da kein Medikament mehr vorhanden). Der Heilerfolg stellte sich innerhalb von 48 - 60 Stunden ein.

### 1.2 Tierart: Katze

Infektiöse Panleukopänie der Katze. Die Parvovirose der Katze betrifft zwar alle Altersstufen, vornehmlich jedoch Jungtiere. Sie verläuft unter dem klinischen Bild einer Gastroenteritis mit ausgeprägter Exsikkose infolge Elektrolytverlustes. Die Mortalitätsrate pendelt zwischen 60 - 90% und tendiert bei jungen Tieren zu 90%. Die zweimalige Anwendung des Leberextraktes in Verbindung mit einem Antibiotikum führte innerhalb von 48 Stunden zum Erfolg (90% bei 157 Fällen).

Nachdem das Präparat nicht mehr zur Verfügung stand, führte die Behandlung mit dem Antibiotikum und einem spezifischen Antiserum nur zu einer Heilungsquote von 60% Die Heilungsquote bei Jungtieren betrug ca. 40%.

### 1.3 Tierart: Rind

Es wurde an Rinderpapillomatose erkrankte Jungtiere behandelt. Die Rinderpapillomatose, verursacht durch DNA-Virus der Papovagruppe tritt besonders in Jungtierbeständen auf. Die antivirale Wirkung des Therapeutikums war bei Rindern besonders deutlich, ablesbar an der Schrumpfung, Nekrotisierung und dem Verschwinden von tumorösen Hautveränderungen. Die gefundene Wirkung ist um so beachtlicher als das verwendete Ersatzmedikament nur eine dem beschriebenen Gesamtextrakt vergleichbare Menge von Wirksubstanz von 20 % aufwies.

### 2. Infektion mit RNA-Viren

### 2.1 Tierart: Katze

Katzenpathogen sind zwei Unterfamilien der Retrovireneinmal das feline Leukämievirus (FeLV) und das feline Immundefizienzvirus (FIV). Beide Krankheitsbilder ähneln sich, zumal beide Viren immunsupressiv sind.

Die Therapie der Erkrankungen FeLV und FIV wird gemeinsam abgehandelt, da zum Zeitpunkt der Behandlung keine deutliche Differenzierung möglich war und in therapeutischer Hinsicht keine Unterschiede bestanden. Der Krankheitsprozess verläuft chronisch. Akut erkrankte Tiere kamen deshalb nicht zur Behandlung. Die Therapie ist naturgemäß langwierig und das Behandlungschema ist differenzierter.

Die Therapie - zwei Injektionen (durchschnittlich 0,6 ml pro Tier) im Ein-Tage-Intervall, dann wöchentlich zwei Injektionen, ab dritter Woche eine Injektion führten in 60% der behandelten 12 Fälle zur Heilung. Alle Tiere wurden auch mit infauster Prognose behandelt.

Bei der üblichen Therapie kommt es trotz teilweiser, vorübergehender Besserung zu keiner Heilung. Ein unterschiedlich langes Siechtum führt immer zum Tode.

### 2.2 Tierart: Schwein

RNA-Infektion mit Schweinepestvirus

Die frühzeitige Behandlung zweier an akuter Schweinepest erkrankter Schweine führte nach zwei Injektionen innerhalb von 60 Stunden zur Heilung.

### 3. Fallstudie zu "generalisierter Rinderpapillomatose" - "Papova-Virusinfektion" in einem Jungrinderbestand in 1993

### Protokoll:

Krankheitsverlauf und Therapie bei 3 Jungrindern der Schwarzbunten Niederungsviehrasse. Alter 1 - 1 1/2 Jahre. Besitzer: Jakob Kadel, Södel Therapiebeginn: 27.4.93

### Probanten:

- Rind 1 und 3 -: jeweils intramuskuläre Injektion
- Rind 2 -: lokale Infiltration eines Papilloms

### Krankheitsverlauf und Therapie:

Rind 1: Schwarzbunt, 1 3/4 Jahre alt, guter Allgemeinzustand

Status Präs.: Insgesamt 10 Papillome über Körper verteilt, davon 7 im Stadium der Proliferation: fleischige, leicht rosa getönte, unbehaarte, runde, auf unterschiedlichem Stiel sitzende tumoröse Veränderungen = Papillome.

Im Einzelnen: linker Hals- und Brustbereich: 3 Papillome, fleischig, kleinkindfaustgroß. linke Vorderextremität: volarseitig 2 Papillome, kartoffelgroß, fleischig, ein kastaniengroßes Papillom, fleischig, auf volarem Metakarpus

rechte Vorderextremität: ein kastaniengroßer, fleischiger Tumor,
Karpalgelenkbereich, dorsomedial
rechte Hinterhand: 3 Papillome größer und in fortgeschrittenerem Stadium (dunkle Oberfläche, zerklüftet)

**Rind 3:** Schwarzbunt, 1 1/2 Jahre, guter Allgemeinzustand

Status Präs.: Insgesamt 3 Papillome, 2 im Rücken, 1 im Halsbereich. Alle in fortgeschrittenerem Entwicklungsstadium - dunkle, zerklüftete, strahlenförmig sich verzweigende Wucherungen.

**Rind 2:** Schwarzbunt, 15 Monate, guter Allgemeinzustand

Status Präs.: 1 Papillome, linke Hinterhand, medialer, oberer Tarsalgelenkbereich. 5-Markstück großer, rosafarbener, fleischiger Knoten.

### Therapie:

Mit Spezialkonzentrat (spz) mit maximal 20% der Stammlösung. Beginn: 27.4.93.

Rind 1: je 6,0 spz i.m. am 27./28./30.4., 3./4.5.93.

Nach 5 Injektionen, gut eine Woche nach Therapiebeginn, am 6.5.93 folgender Zustand:
rechte Hinterhand: 3 Papillome bis auf kleine Stielreste abgefallen.
linker Hals und Brustseite: alle 3 Papillome im Stadium der Rück- und Umbildung = welker, dunkler zerklüftet.
linke Vorderhand (volar): 2 Papillome in deutlicher Rückbildung = Demarkierung auffallend dunkler, von brüchiger Konsistenz, rosettenartige Wucherungen mit deutlich geringerem Turgor.

Unterhalb Karpalgelenk: Kleines Papillom in Rückbildung.

Rechte Vorhand: Kleines Papillom noch mehr fleischig, geringe Rückbildungstendenz.

### Anschlusstherapie:

Ab 6.5.93 noch insgesamt 9 Injektionen, je 7,0 spz i.m. im zweitägigen Intervall.

### Weiterer Therapieverlauf:

8.5.93 Papillome welker - linke Vorhand (volar): Zwillingsgeschwulst vergrößert, mit deutlicher Lappenbildung.

11.5.93 (2 Wochen nach Therapiebeginn): Papillom Buggelenk linksseitig abgefallen, insgesamt fortschreitender Umbau in Richtung Morphologie des Spätstadiums der Papillomatose.

17.5.93 (3 Wochen nach Therapiebeginn): rechte Vorhand: Karpalgelenktumor abgefallen.

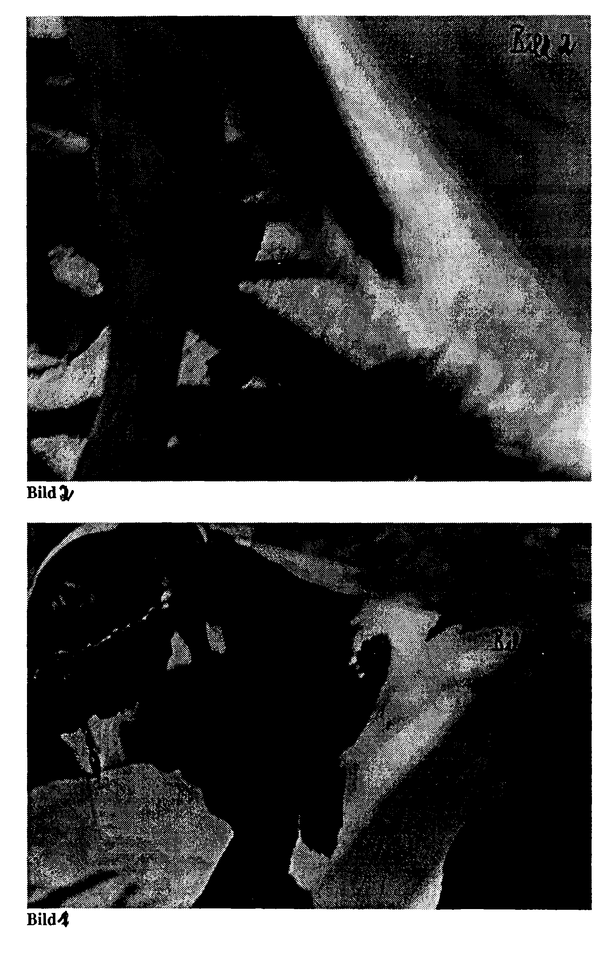

24.5.93 (fotografisch dokumentiert, Bild 1 und 2): Vier Wochen nach Therapiebeginn auffällige Rückbildung aller Papillome - linke Vorhand volarer Karpalbereich ein Teil der Zwillingsgeschwulst abgefallen (kleiner Stielrest).

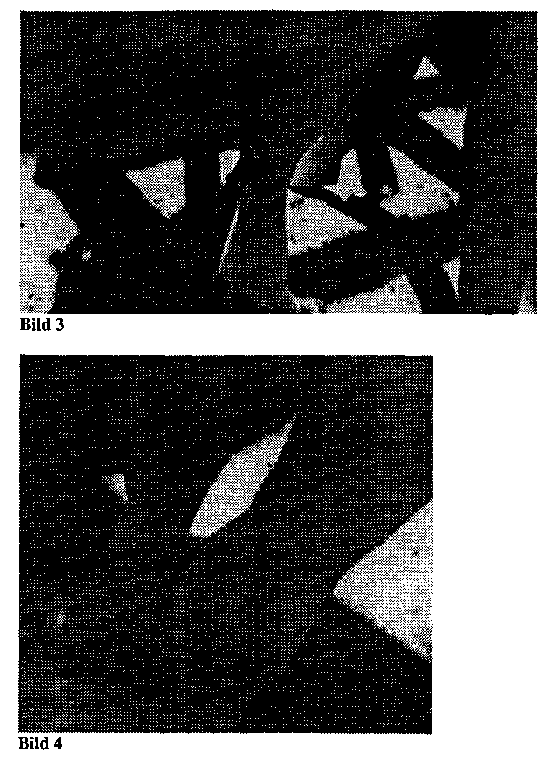

2.6.93 (5 Wochen nach Therapiebeginn, Bild 3 und 4): Insgesamt nur noch 2 stark rückgebildete Papillome.
Bild 3 = linke Vorhand, volarer Karpalgelenkbereich
Bild 4 = völlige Abheilung, kleine haarlose Stelle auf linksseitigem, dorsalem Karpalgelenkbereich.

16.6.93 (7 Woche nach Therapiebeginn): Beide restliche Papillome kurz vor endgültiger Demarkation (völlig abgestorben).

### Rind 3

Therapie: 3 x je 5,0 spz i.m. am 27./30.4. und 3.5.93

### Therapieverlauf:

Nach gut einer Woche (6.5.93) fortschreitender Zerfall der Papillome.

15.5.93: ein Papillom leicht durch Torsion zu entfernen.

19.5.93: Beide restliche Papillome durch Torsion entfernt (weitgehend demarkiert, 3 Wochen nach Therapiebeginn)

### Rind 2

**Therapie: Einzeltumor zunächst lokale Infiltration mit je 2,0 spz.**

27./30.4, 3./4./6.5.93: Bis zum 8.5 keine exakt zu beobachtende Veränderungen festzustellen.

Ab 13.5.93 Umstellung auf i.m.-Therapie mit 5,0 spz.

15./17./19./25./28.5.93: je 7,0 spz i.m. (insgesamt 6 Injektionen).

### Therapieverlauf:

17.5.93: geringe Umfangsvermehrung des Papilloms, zentrale Dunklerfärbung, Abnahme des Turgors.

24.5.93: Papillom erscheint welker.

Mitte Juni (4 Wochen nach Beginn der i.m.-Therapie): Tumor abgefallen.

### Wertung der Therapieergebnisse:

Trotz des möglichen Einwandes einer beschränkten Aussagefähigkeit infolge der geringen Zahl an Probanden muss festgehalten werden:
1. Bei allen drei Probanden stellte sich ein Therapieerfolg im vertretbaren zeitlichen Rahmen ein.
2. Erwartungsgemäß war die I.M-Injektion die einzig wirksame Applikationsform. Die Umstände des Therapieverlaufs bei Rind 2 zeigen dies deutlich. Desweiteren ist daraus zu entnehmen, dass lokale Mechanismen einen Therapieerfolg nicht bewirken können. Auch sei in diesem Zusammenhang darauf hingewiesen, dass Vakzinen aus Papillom-Material eine nur begrenzte Wirkung besitzen, die lediglich zu einer Verkürzung des Krankheitsverlaufs von 1 - 2 Monaten des natürlichen Krankheitsverlaufes von 3 - 6 Monaten führt. Quelle: Buiatrik, Bd. II, Ausg. 1996, Verlag Schaper Hannover, S. 81.
3. Während des Heilungsverlaufs infolge der Therapie zeigen sich alle Stadien der Abheilung entsprechend dem natürlichen Verlauf.
4. Während der Behandlung kam es zu keinen Nebenwirkungen oder irgendwelchen Auswirkungen auf den Gesundheitszustand der Rinder.
5. Ab dem Zeitpunkt der beginnenden Rückbildung der Tumoren bewirkt ein Weiterführung der Therapie keine wesentliche Beschleunigung des Abbaus der Papillome. Erklärt sich zwingend mit der Tatsache, dass die Abbauvorgänge weitgehend biologisch determiniert sind. Vergleichbar z.B. mit dem Verlauf einer klassischen kruppösen Pneumonie.

## Patentansprüche

1. Verwendung eines Leberextraktes und/oder Fraktionen desselben als Virustherapeutikum (universelles Immunstimulans).

2. Verwendung eines Leberextraktes und/oder Fraktionen desselben als virales Antiinfektiosum.

3. Verwendung eines Leberextraktes und/oder Fraktionen desselben zur Therapie von durch Prionen induzierten Erkrankungen.

4. Verwendung nach Anspruch 3, dadurch gekennzeichnet, dass das Leberextrakt peridural appliziert wird.

5. Leberextrakt für die Verwendungen nach einem der vorangehenden Ansprüche, dadurch gekennzeichnet, dass es sich um ein Hydrolysat handelt.

6. Leberextrakt nach einem der vorangehenden Ansprüche, dadurch gekennzeichnet, dass es sich um ein Lebervollextrakt handelt.

7. Leberextrakt nach zumindest einem der vorangehenden Ansprüche, dadurch gekennzeichnet, dass es einen Konservierungsstoff wie m-Kresol enthält.

8. Leberextrakt nach zumindest einem der vorangehenden Ansprüche, dadurch gekennzeichnet, dass es in homöopathischen Dilutionen für spezielle Indikationen (peridurale Injektion) verwendet wird.
